# EUROPEAN PATENT APPLICATION

(11) **EP 3 278 815 A1**
(43) Date of publication of application: **07.02.2018**
(21) Application number: 16771436.9
(22) Date of filing: 12.02.2016
(51) Int. Cl.: A61K 47/32, A61K 47/36, A61K 9/06, A61K 31/19, A61K 31/195, A61K 31/455, A61K 33/38, A61L 15/28, A61P 17/02

(54) **HAEMOSTATIC AND WOUND HEALING MEDICINE**

(30) Priority: 02.04.2015 EA 201500358
(71) Applicant: Limited Liability Company "Tectum", Nizhny Novgorod 603950 (RU)
(72) Inventor: KORYAGIN, Alexandr Sergeevich, Nizhny Novgorod 603002 (RU); MOCHALOVA, Alla Evgenievna, Nizhny Novgorod 603123 (RU); SMIRNOVA, Larisa Alexandrovna, Nizhny Novgorod 603087 (RU); APRYATINA, Kristina Viktorovna, Voznesensky r-n Nizhegorodskaya obl. (RU); GRIGORIEVA, Elena Nikolaevna, Nizhny Novgorod 603087 (RU)
(74) Representative: Chimini, Francesco
(86) International application number: PCT/EA2016/000001
(87) International publication number: WO 2016/155749

(57) **Abstract**

The present invention refers to medicine, namely hemostatic and wound healing agents, and can be used to stop bleeding of various severity, including venous, as well as to heal wounds and cuts obtained as a result of mechanical action on the epidermal and muscular layers of tissues. For this, a hemostatic agent containing an active gelling agent based on chitosan and / or its derivatives is used, characterized in that it contains chitosan, its salts and / or its derivatives in the form of a gel having a three-dimensional crosslinked structure as a polymer matrix and components of natural thrombus formation in the form of organic calcium compounds, organic acid and water coordinated on this matrix at a certain ratio of components.

The technical result of the invention consists in accelerating the arrest of bleeding of various severity and creating an antibacterial effect.

## Description

### TECHNICAL FIELD

The present invention refers to medicine, namely hemostatic and wound healing agents, and can be used to stop bleeding of various severity, including venous, as well as to heal wounds and cuts obtained as a result of mechanical action on the epidermal and muscular layers of tissues.

### BACKGROUND

Currently, various hemostatic and wound-healing agent are actively being developed in medicine, veterinary medicine, and pharmaceutics.

At the moment, such tools as bandages, hemostatic sponges, hemostatic powders are used in first aid. The main drawback of all available tools is that they need to be removed from the wound after application, thereby re-inflicting mechanical damage. That is why creating an atraumatic hemostatic agent, biocompatible with living tissues of the human body that simulates the natural hemostasis, simultaneously possessing wound healing and bactericidal properties poses a relevant challenge.

There is a prior healing agent, which is protected by patent RU2271814, IPC A61 K31 /722, A61 K31 / 785, A61 K31 / 136, A61 K31 / 375, A61 K38 / 43, A61 P17 / 02, publ. on 20.03.2006.

The healing agent contains chitosan, a vitamin, an antiseptic and an anesthetic and, in addition, enzymes. Powdered chitosan is a preparation of natural origin, preferably produced from the carapace of crabs. Vitamin C or the ascorbic acid is used as a vitamin preparation, while polyalkylene guanides from a number of phosphopagues and pyromecaines are chosen as antiseptic. The anesthetic is chosen from a series of trimecaine and pyromecaine, an enzyme proteolytic drug is chosen from a range of pepsin and collagenases.

The disadvantage of the prior agent is that this agent does not have hemostatic properties, does not contain hemostatic components, and can only be used as a wound-healing agent. This prior agent uses dioxidine as a bactericidal component. A significant disadvantage of dioxidine is its mutagenic activity, embryotoxicity and the possibility of damaging the cortical layer of the adrenal glands. This effect is dose-dependent. Obviously, an overdose of dioxin in humans can cause side effects associated with impaired glucocorticoid synthesis, which requires the immediate discontinuation of the drug and appropriate hormone replacement therapy.

There is a prior hemostatic, which is protected by patent RU2519220, IPC A61 K31 / 722, A61 K31 / 155, A61 L15 / 2, A61 L26 / 00, A61 P7 / 04, published on 04/10/2014, designed to stop massive bleeding. The agent contains 75 to 95 wt% of chitosan salt from a series of polydisperse powders of hydrochloride, hydrobromide, formate, acetate, succinate, citrate, glycollate or chitosan lactate and 4-20 wt% of polyhexamethyleneguanidine hydrochloride. The chitosan salt and polyhexamethylene guanidine hydrochloride are covalently cross-linked by a 1-5 wt% of polyfunctional compound of glycidyl. The chitosan salt is chosen with an average particle size of 0.2+2.0 mm, the degree of deacetylation of chitosan 0.75+0.95, with a molecular mass of 10+500 kDa. The agent uses diglycidyl ether of butandiol, di- or triethylene glycol or propylene glycol, oligoethylene oxide, as well as triglycidyl ethers of glycerol or trimethylol propane as polyfunctional compound of glycidyl ethers.

A disadvantage of the prior agent is that the agent is a powdered composition. Dry composition, requires swelling and mechanical action (pressure) to provide the desired effect, which can result in incomplete filling of the wound and painful effects when applied. The powdery nature of the components may lead to their uneven distribution. Compounds from a series of diglycidyl ethers are used as crosslinking components. These compounds are unstable, their hydrolysis is observed during the storage, which limits the duration of the agent.

The closest to the claimed invention in terms of technical essence selected as a prototype is a hemostatic burn and wound-healing composition protected by patent RU2526183, IPC A61 K47 / 36, A61 K9 / 06, A61 K31 / 72, A61 F13 / 02, publ.August 20, 2014

The composition is produced in the form of a hydrogel and contains an active gelling agent, a plasticizer, active and auxiliary components, namely a water-soluble heteropolymer of a chitosonium salt in the amount of 1,0 to 10.0 wt%, a dexpanthenol and/or 2-allyloxyethanol substance in the amount of 1,0 to 10.0 wt%, immobilized medical substances of aminocaproic or tranexamic acid in the amount of 0.1 to 5.0 wt% and calcium chloride (CaCl₂) in the amount of 0.05 to 2.0 wt%, immobilized medicinal substances of lidocaine or anilocaine in the amount of 0. 1 to 5.0 wt% and chlorhexidine in the amount of 0.005 to 0. 1 wt% and water. The disadvantage of the known composition is that 2-allyloxyethanol and chlorhexidine are capable of causing an allergic reaction including the anaphylactic shock. Chlorhexidine has a pronounced irritant effect on the skin and eyes. In addition, one of the main components of the composition is the inorganic compound CaCl₂. The inorganic compound CaCl₂ dissociates into Ca2⁺ and CL⁻ ions, and as a result of rapid decay, the substance is able to rapidly penetrate into the cell, which can lead to a cytotoxic effect and hyperosmosis. Dissociated into ions, the compound is quickly disposed of and, as a consequence, quickly removed from the wound surface area. As a result, the action time of this drug is very short, which increases the time of hemostasis. In addition, the gel does not have a spatial structured mesh in this structure of the composition, since chitosan is represented as isolated chains of chitosan macromolecules chemically unrelated to each other. Therefore, the composition cannot model fragments of the membrane of blood cells and the walls of vessels, which have a three-dimensional matrix structure that provides a natural process of blood coagulation. A short period of storage of the drug is due to spontaneous hydrolysis of chitosan chains in acid media.

### DISCLOSURE OF THE INVENTION

The aim of the present invention is to create an atraumatic, biocompatible with living tissues of the human body, hemostatic agent that operates on the basis of natural hemostasis, and simultaneously possesses bactericidal and wound healing properties and a shelf life of at least 3 years.

The technical result of the invention consists in accelerating the arrest of bleeding of various severity and creating an antibacterial effect.

Said technical result is achieved by developing and obtaining a hemostatic and wound healing agent containing an active gelling agent based on chitosan and / or derivatives thereof, which is unique due to the fact that it comprises chitosan, its salts and / or its derivatives in the form of a gel with a three-dimensional crosslinked structure as polymer matrix and components of natural thrombus formation in the form of organic calcium compounds, organic acid and water coordinated on such matrix at the following ratio of the components, %wt:
chitosan, its salts and / or its derivatives - 0.2-30;
organic compounds of calcium - 0,01-10;
organic acid - 0,1-10;
water - the rest,
in addition, block-and graft copolymers such as chitosan-polyvinylpyrrolidone, chitosan-poly-2-hydroxyethyl methacrylate / poly-2-hydroxypropyl methacrylate, chitosan-polylactide, chitosan-polyglycolide are used as chitosan derivatives, and aspartic acid, succinic acid or nicotinic acid are used as an organic acid.

The agent also contains calcium glycerophosphate, calcium gluconate, calcium succinate or calcium nicotinate as components of natural thrombus formation in the form of derivative organic compounds of calcium complexed with chitosan.

In addition, aminocaproic acid is introduced into the composition as inhibitor of fibrinolysis. To obtain an antibacterial effect, silver nanoparticles are introduced into the composition of the agent.

The abovementioned and other aspects and advantages of the present invention are disclosed in the following detailed description thereof.

### DETAILED DISCLOSURE OF THE INVENTION

The fact that the preparation contains chitosan, its salts and / or its derivatives, in particular block copolymers, in the form of a gel with a three-dimensional crosslinked structure in the form of a polymer matrix and coordinated natural components of the thrombus formation, makes it possible to model fragments of the membrane of blood cells and walls of the vessels with a three-dimensional matrix structure, which in the presence of calcium ions provides a natural process of blood coagulation.

A gel with a three-dimensional structure is formed during the modification of chitosan, either due to condensation processes of bifunctional monomers with reactive groups, or during radical polymerization of bifunctional vinyl monomers. This complex of components fosters the process of blood coagulation.

The structure and, consequently, the sorption-diffusion, elastic-plastic, optical and other parameters of the composition are largely determined by the way it is formed and its modifications (chemical, physical-chemical). The chitosan macromolecule has a heterochain structure and is constructed from the units of D-glucosamine residues that determine the degree of deacetylation (DD) of the polysaccharide and a small amount of D-acetyl-D-glucosamine bound by P-1,4-glycosidic bonds. Polyfunctionality of this polymer leads to the fact that, depending on the method of preparation, chitosan in this composition can have two chemical forms: salt (C-) and base (O-), differing in physicochemical and biochemical characteristics. The heat treatment, the introduction of biologically active substances in the polymer matrix, the processing of crosslinking agents, etc, also have a significant effect on the properties of the composition. Nevertheless, the composition not only preserves a complex of valuable properties, but as a result of the modification it can significantly improve them and even acquire new useful properties. The objects of this study - materials based on chitosan and its salts, as well as block and graft copolymers of chitosan can be refered to as such compositions. (Mochalova A.E., Nikishchenkova L.V., Smirnova N.N., Smirnova L.A. Thermodynamic properties of hydrogels based on chitosan in the range from T-> 0 to 350 K. // High-molecular compounds, Biology-2007 series - T. 49, Issue 2. - P. 371-376.)

The presence of a three-dimensional structure provides for a long-term storage of the composition: the storage time of the product is increased to 3 years.

Hemostatic and wound healing agent is prepared as follows:
An organic acid - 0.1-10% is added into the mixture of chitosan salt and chitosan block-polylactide, salts of chitosan and / or its derivatives, which ensures the formation of a stable gel. The component responsible for natural thrombogenesis in the form of an organic compound - calcium nicotinate, calcium glycerophosphate, calcium gluconate, calcium succinate in an amount of 0.01-10 wt% is added during mixing to the gel based on chitosan and chitosan block polylactide, the salt of chitosan and / or its derivatives in the amount of 0.2-5.0 wt%..

In the mixing stage of the starting components, various additives such as antibacterial agents can be added to the reaction mixture. Such antibacterial agents can be silver nanoparticles in the amount of 0.0001 -1.0 wt%, analgesic components, proteolytic enzymes, antioxidants, stimulants for regeneration of wounds of various etiologies (for example, aminocaproic acid as an inhibitor of fibrinolysis, in an amount of 0.1-5.0 wt%, and other medicaments, which can help heal wounds and burns.

### Example 1.

The mixture of chitosan salt and chitosan block polylactide is supplemented with aspartic acid, which provides the formation of a stable gel. A gel based on chitosan and chitosan block-polylactide (5.0 wt%) is supplemented with a component for natural thrombus formation in the form of an organic calcium compound, calcium glycerophosphate, in an amount of 0.2 wt% and made up to 100% with water.

### Examples 2-7 were conducted in a manner similar to Example 1,

The data is summarized in the table.

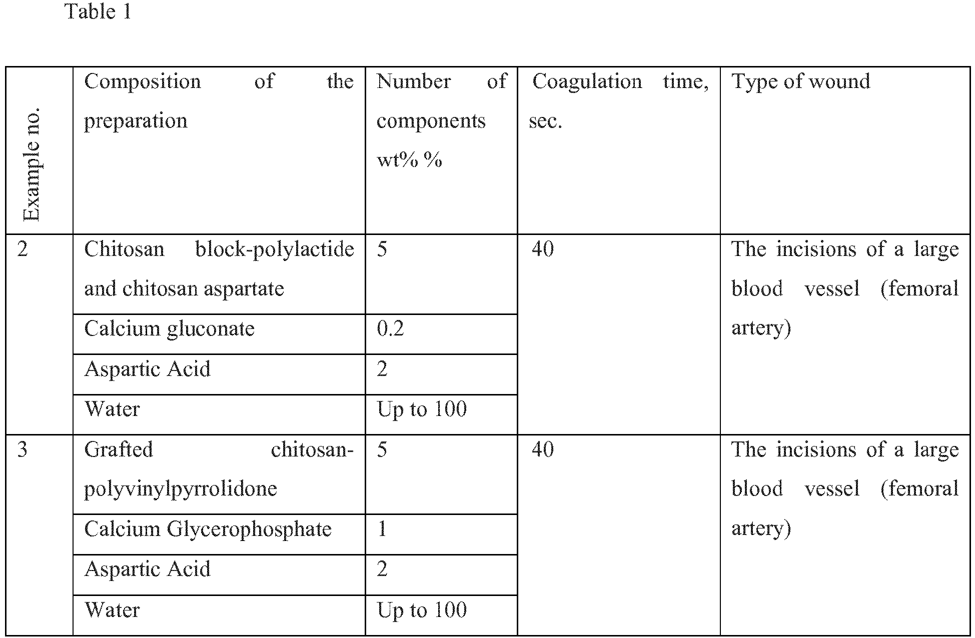

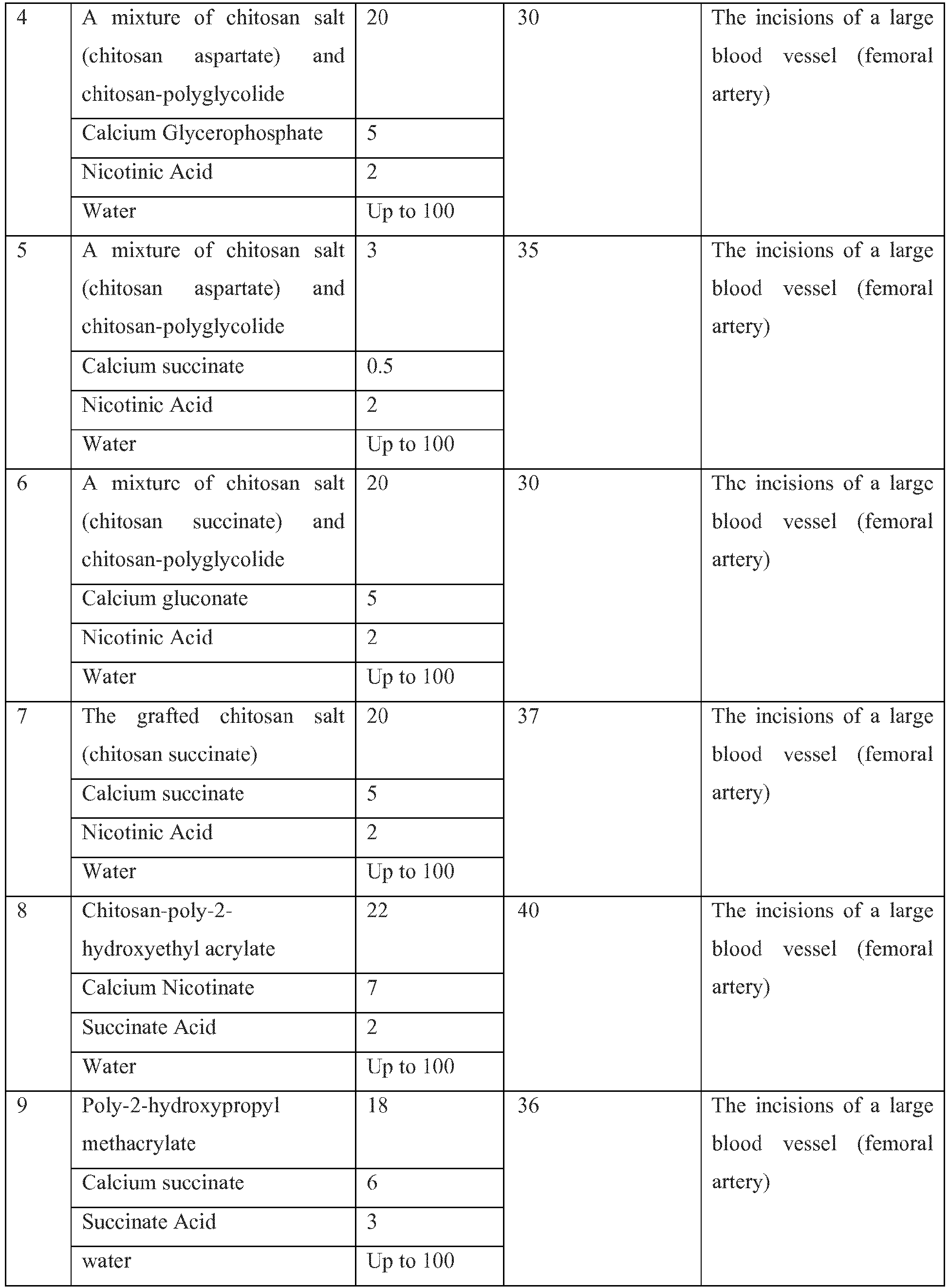

An increase in the amount of chitosan of more than 30 wt% will lead to a substantial structuring of the system, and a decrease of less than 0.2 wt% will lead to a difficulty in forming a three-dimensional structure.

An increase in the number of components of natural thrombus formation in the form of organic compounds of Ca by more than 10% will not lead to further improvement in the properties of the composition and will not increase the speed of coagulation, and the amount of organic compounds of Ca less than 0.01% will substantially reduce the effectiveness of the hemostatic effect.

An increase in the number of inhibitors of fibrinolysis of more than 10 wt% and a decrease of less than 0.1 wt% will lead to a decrease in the rate of thrombus formation.

An increase in the amount of silver nanoparticles greater than 1 wt% will lead to the appearance of a toxic effect of silver on the body cells. A large amount of AgNO₃ increases the healing time. Reducing the amount of silver nanoparticles by less than 0.01 wt% will lead to the disappearance of the bactericidal effect of the composition; it will continue exhibiting only bacteriostatic properties.

The organic acid can be amber, nicotinic or aspartic; it does not affect the properties of the composition. Any of these acids is administered in the amount sufficient to form a uniform homogeneous system and normalize the pH to match the interior milieu (pH does not exceed 5.2-5.4).

The study of clotting time was carried out as follows.

### In vitro tests on the watch glass.

3-4 drops of citrated blood from laboratory animals were applied onto a sterile watch glass. Citrate blood is blood that contains sodium citrate as an anticoagulant.

The blood was covered with 100 µl of the hemostatic agent. Immediate formation of microscopic blood clots was observed throughout the mixture.

### In vitro tests on hemocoagulographer N-331

The study used blood from nonlinear white rats. The total clotting time with the addition of a hemostatic agent was studied. To do this, 200 µl of blood was extracted into a cuvette from the sublingual vein of experimental animals and 100 µl of a hemostatic agent was added. After that, the instrument readings were taken.

The average time of the start of coagulation is 23 s.

The average time of the end of coagulation is 43 s.

Intact blood. The average time of the start of coagulation is 85 s. The time of the end of coagulation is 153 s.

### In vivo tests in experimental animals

In vivo tests were performed on pre-anesthetized non-linear white rats.

Preliminary study of coagulation time of intact blood was conducted. For this purpose, a skin flap from the left hip approximately 2x2 cm in size was prepared from each animal exposing the femoral vein, which was then incised; the time of hemorrhage was registered using the stopwatch. The formation of a thrombus at the site of the incision and the cessation of the blood flow was considered the time of the hemostasis.

The average hemostasis time of intact blood is 100 s.

Further, the time of hemostasis after application a hemostatic agent to the cut was examined. The agent was applied immediately after the vein incision was performed. The time of blood clotting was registered.

The average time of hemostasis after application of the agent to the incision is 40 seconds.

Blood clotting time reached 30-40 seconds through adsorption of platelet fixation on chitosan.

The tests of the claimed agent were carried out at the Department of Physiology and Anatomy of Animals and Human of the Faculty of Biology of Lobachevsky State University of Nizhny Novgorod. Tests of this agent began 2 years ago. Ten nonlinear rats were used in each experiment, 5 intact (norm) and 5 experimental ones. Each animal was previously anesthetized with diethyl ether. A 2x2 cm thick skin flap was prepared from each animal, exposing a large femoral vessel. Then neat cuts were made to cause bleeding, after which a hemostatic agent was applied to the wound of the animal. The applied gel quickly stopped the bleeding with the formation of a surface film on the wound. Further observation of experimental animals showed that the formation of the epithelial cover on the wounds treated with the solution occurred without infection of the wound 2 times faster than in the control animals.

To test the effectiveness of the hemostatic agent and the optimality of its composition, a number of control tests were carried out, in which one of the key components was excluded from the formulation, and the effect of its absence in the composition on the coagulation time was studied:
- the clotting time of the blood when aqueous solutions of calcium was applied to the bleeding incision of veins was ∼ 150 s;
- the clotting time of the blood when the solutions of chitosan salts were applied to the bleeding incision of the vein was ∼ 84 s;
- the clotting time when solutions of calcium salts in an aqueous solution of acid were applied to a bleeding vein was - 100 s.

When the integrity of the skin and tissues is compromised, wound surface infection often occurs. Therefore, the development of products with effective hemostatic properties requires the introduction of components that provide a high bactericidal effect. To this end, silver nanoparticles were introduced into the composition. Table 2 shows the results of bactericidal tests

**Table 2**

| | Zone of inhibition of bacterial growth, R (mm) | | |
|---|---|---|---|
| | Escherichia coli | Pseudomonas aeruginosa | Staphylococcus aureus |
| Bactericidal effect of compositions with silver nanoparticles, with nanoparticle content of 0.006 wt.% | 6 | 4 | 3 |
| Comparison composition that does not contain silver nanoparticles | 0.5 | 0.5 | 0.5 |

Thus, in comparison with the prototype, the proposed hemostatic and wound-healing agent, when in contact with the source of bleeding, influences the hemostasis and accelerates the natural process of blood clotting. Blood clotting time reached 30-40 seconds through adsorption of platelet fixation on chitosan.

In addition, the claimed agent provides elasticity, adsorption, and deep congruence throughout the wound surface. An additional effect from the use of the claimed agent is the transparency of the composition in the visible and UV wavelengths, which allows additional treatment with UV radiation.

The above distinctive essential features are necessary and sufficient and manifest new properties in the claimed combination that are not clearly derived from the state of the art in the field and are not obvious to an expert. An identical combination of features was not found in the analyzed literature

The experts may find evident that there can be other embodiments of the invention that do not alter its essence, as it is disclosed herein. Accordingly, the invention should be considered limited in scope only by the following claims.

## Claims

1. A hemostatic and wound healing agent comprising an active gelling agent based on chitosan and/or its derivatives, unique in that it comprises chitosan, its salts and/or its derivatives in the form of a gel having a three-dimensional crosslinked structure as a polymer matrix and components of natural thrombus formation in the form of organic calcium compounds, organic acid and water coordinated on this matrix at the following ratio of components, %wt:
chitosan, its salts and/or its derivatives - 0.2-30;
organic compounds of calcium - 0,01-10;
organic acid - 0,1-10;
water - the rest,
in addition, block-and graft copolymers such as chitosan-polyvinylpyrrolidone, chitosan-poly-2-hydroxyethyl methacrylate/poly-2-hydroxypropyl methacrylate, chitosan-polylactide, chitosan-polyglycolide are used as chitosan derivatives, and aspartic acid, succinic acid or nicotinic acid are used as an organic acid.

2. The agent of claim 1, which contains calcium glycerophosphate, calcium gluconate, calcium succinate or calcium nicotinate as components of natural thrombus formation, coordinated with chitosan, its salts and / or its derivatives of organic calcium compounds.

3. The agent of claim 1, **characterized in that** it additionaly comprises a fibrinolysis inhibitor.

4. The agent of claim 3, **characterized in that** the fibrinolysis inhibitor is an aminocaproic acid.

5. The agent of claim 1, **characterized in that** it additionally contains silver nanoparticles.
